# NEW EUROPEAN PATENT SPECIFICATION

(11) **EP 1 322 256 B2**
(45) Date of publication and mention of the opposition decision: **21.12.2011**
(45) Mention of the grant of the patent: 28.12.2005
(21) Application number: 01971318.9
(22) Date of filing: 24.09.2001
(51) Int. Cl.: A61F 2/06

(54) **INTRAVASCULAR STENT APPARATUS**
INTRAVASKULÄRER STENT
DISPOSITIF D'EXTENSEUR INTRAVASCULAIRE

(30) Priority: 25.09.2000 US 235180 P
(43) Date of publication of application: 02.07.2003
(60) Divisional application: 05019033.9
(73) Proprietor: Boston Scientific Scimed, Inc., Maple Grove, MN 55311 (US)
(72) Inventor: Jang, G. David, Redlands, CA 92374 (US)
(74) Representative: Hermann, Gerhard
(86) International application number: PCT/US2001/029793
(87) International publication number: WO 2002/026164

(56) References cited:
- EP-A- 0 980 694
- EP-A2- 1 309 293
- EP-B1- 1 318 772
- WO-A1-02/24109
- WO-A1-98/48735
- WO-A1-99/49928
- US-A- 6 039 756
- US-A- 6 113 627

## Description

This invention relates to intravascular stents in general, and more particularly to intracoronary stents.

### Description of the Related Art:

Intracoronary stents provide intraluminal scaffolding support of the vascular wall after percutaneous angioplasty in which the balloon catheter is used to expand the stenotic vascular lesion. In both the delivery phase and the deployed phase, there are numerous performance factors that can characterize the overall clinical performance of a stent and can be improved.

By the year 2000, the percutaneous balloon angioplasty and stent implant procedures have become the dominant non-surgical revascularization method of the atherosclerotic stenosis, or obstruction, of the vascular lumen, and particularly in the coronary vascular system of the heart. With balloon angioplasty alone and without stents, the restenosis rate after angioplasty has been as high as 25-45% in the first time coronary cases. With stents after balloon angioplasty, the restenosis rate has been reduced significantly. Even so, the restenosis rate after stent implantation is reported to be 15-25% range in coronary arteries, depending on the condition of the stented vessel or the specific stent. An ideal coronary stent is still elusive in the current state of the art commercial products.

Some of the best selling current, second generation, stents can be divided into two categories. One category is a stent with high flexibility and the other category has full vessel coverage. The flexible stents generally have poor vessel coverage, tissue prolapse, rough surface modulation and increased restenosis rate. On the other hand, a stent with good vessel coverage in the current state of art may not be flexible enough for easy delivery and for highly efficient procedures. This means that a stent with good flexibility and good vessel coverage remains as the unfulfilled gold standard.

To further reduce the restenosis rate after stent implant, numerous means have been tried including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient, and costly. Brachytherapy is a radioactive device and a radiation therapy specialist from another department has to be involved with the interventional cardiologist in the cardiac catheterization laboratory. The laser and atherectomy devices proved to be marginally useful with added costs.

Local drug therapy appears to be a very promising method for the future, as better pharmaceutical, chemical, or biogenetic agents are developed and became available. Some research data, both from animal tests and human clinical studies indicate evidence of some suppression of restenosis after stent implantation when certain growth blocking pharmaceutical agents coat the stent. In other instances, it has been speculated that certain surface modifying materials coated on the surface of the stent may be beneficial, alone or in combination with growth suppressing agents, in reducing the restenosis rate. In either instance, a drug or substance should be locally attached or coated on the stent in sufficient amounts. However, attaching or coating a sufficient amount of a substance or drug on the coronary stent may not be an easy proposition, because coating enough volume of the drug on the small surface area of a stent is a challenging task. If and when stent coating becomes practical, a good stent can still have better outcomes than a poorly designed stent when used with substance coating.

A stent is a scaffolding device. When delivered to a remote vessel location via percutaneous approach it can be deployed by expanding the device inside a vessel. The vessel can have a very small caliber and sometimes has a very tortuous anatomy. When a stent is deployed, the stent should have a good radial strength, good vessel coverage, a good internal surface modulation without tulips (i.e., sharp metal loop projections that resemble fish scale phenomena), optimal vessel conformability, a low metal fraction, and so forth. If the stent is stiff and non-flexible, it can be very difficult to deliver to an intended lesion site inside a vessel. Easy delivery of a stent is aided by good flexibility of the stent in combination with the delivery balloon, a smooth surface modulation without or minimizing tulips and a degree of radiopacity. A good stent should have a combination of features for delivery and deployment.

Although there are countless variations of vascular stent designs today, few have these desired stent features both in the delivery phase and in the post-delivery phase. Today's top selling stents in the market can have undesirable characteristics, either in the delivery phase or in the deployed phase of the stent life cycle. For example, some stents may have flexibility, but lack vessel coverage or surface modulations both in delivery and deployed phases. Some stents may have good vessel coverage and surface modulations, but lack flexibility.

Vascular stents, which are designed to be delivered to vessel sites via percutaneous approach, can have two elements. The first element is the expansion strut that expands circumferentially to provide the scaffolding radial force against a possible collapsing force of the vessel wall. The second element is the connecting strut that can link the expansion struts along the longitudinal axis of the stent, giving articulation or flexibility to the stent. The particular combination of expansion struts and connecting struts generally form various cells, depending on the specific configuration and shape of the expansion and connecting struts. If a cell is too large, the vessel wall support or coverage can be poor and the vessel wall tissue can prolapse through the large cells of the stent net. If the cells are too small, the vessel wall may be well covered but the metal fraction of the stent can be too high. The metal fraction is a fraction of the total metal surface area of an expanded stent (inside a blood vessel) divided by the total internal vessel wall surface area where the stent is deployed.

Some very flexible stents have very large cell size with poor vessel coverage and tissue prolapse, in addition to poor (inner and/or outer) surface modulation due to large numbers of tulips directed to both ends of the stent.

Most of the current flexible stents are designed to effect flexibility by using fewer or a minimal number of connecting struts, handicapping the vessel coverage, surface modulation and tissue prolapse defects.

On the other hand, a stent that is designed for good vessel coverage and ideal cell size tends to be inflexible when such a stent is being delivered to a vessel lesion. A lack of flexibility during stent delivery is a very critical issue; a stiff stent often cannot be delivered to a needed location inside a blood vessel because such a stent cannot navigate through a tortuous and small vessel lumen.

US 6,113,627 discloses a tubular stent consisting of horizontal expansion struts and contralaterally attached diagonal-connectors. The known tubular stent comprises connecting struts having a proximal portion and a distal portion. The intermediate portion is of a linear configuration.

The underlying problem of the invention is to provide a vascular stent that is very flexible for delivery and has a good vessel coverage when deployed.

The problem is solved by a stent according to claim 1.

### SUMMARY OF THE INVENTION

Various embodiments of a stent include a combination of maximum possible flexibility and conformability in the stent, full vessel coverage with optimal metal fraction, evenly expanding stent struts, excellent radial strength and radiopacity, and smooth surface modulations in both delivery and deployed phases of the stent life cycle. To arrive at these goals, many detailed new innovations are added to the expansion and connecting strut designs of the stent. Expansion strut design is largely responsible for radial strength and radiopacity, while connecting strut design is largely responsible for flexibility and smooth surface modulations. Full vessel coverage and uniform stent expansion are largely from interaction between expansion and connecting struts. Various embodiments of the stent demonstrate a balance among these multiple qualities, using smart expansion struts and flexible connecting struts in a seamlessly integrated stent network.

Various embodiments of the stent are specifically designed to be both very flexible and fully cover vessel surface inside the vascular lumen. The stent can have both characteristics of vessel coverage and flexibility, particularly for coronary use.

Various embodiments of a stent are well designed for both the delivery phase and the deployed phase of the stent life cycle. Both flexibility and good vessel coverage are in a right balance in various embodiments of the stent have. Various embodiments of the stent include certain configurations in expansion and connecting struts of the stent.

### BRIEF DESCRIPTION OF DRAWINGS

Figure 1 shows a side elevation view of an embodiment of a stent, such as a tubular stent.
Figure 2 shows an isometric view of an embodiment of a stent, such as a tubular stent.
Figure 3 shows a cut-open view of an embodiment of a stent. Various expansion columns and connecting strut columns are shown.
Figure 4 shows a magnified view of a middle section of an embodiment of a stent, such as a stent of Figures 1, 2, and 3. Some details are shown of expansion columns.
Figure 5 shows a magnified view of a middle section of an embodiment of a stent, such as a stent of Figures 1, 2, and 3. Some details are shown of connecting strut columns.

### DETAILED DESCRIPTION OF DRAWINGS

Some embodiments of stents can be in a state, such as one or more of a non-expanded state, an expanded state, a crimped state, and a non-crimped state.

Some embodiments of stents can include one or more of a first expansion column, a second expansion column, a third expansion column, a first connecting strut column, and a second connecting strut column.

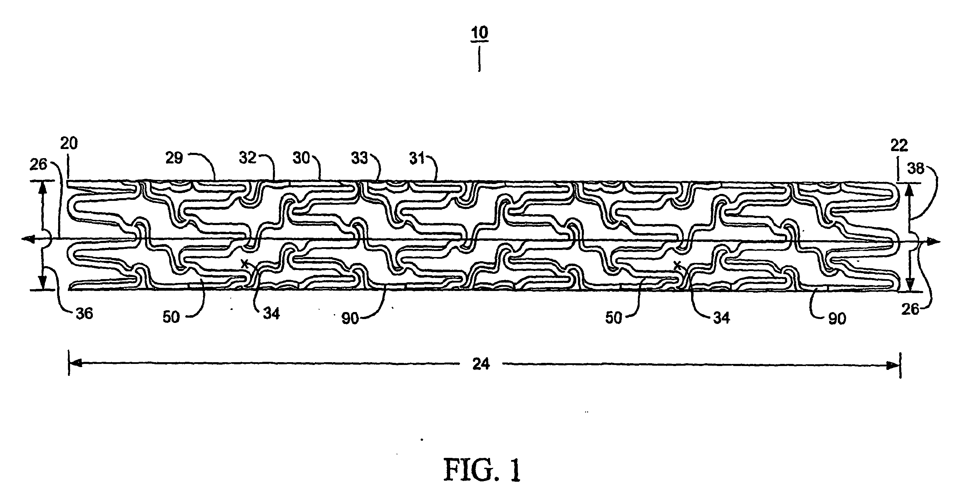

Figure 1 shows an embodiment having a first expansion column 29, a second expansion column 30, a third expansion column 31, a first connecting strut column 32, and a second connecting strut column 33. The first expansion column, the second expansion column, and the third expansion column can include individual expansion struts forming a plurality of expansion strut pairs. In many embodiments of the stent, two adjacent expansion strut pairs share a common strut.

The first connecting strut column and the second connecting strut column include a plurality of individual connecting struts. Each connecting strut has a stair-step geometric configuration with a curvilinear proximal section and a curvilinear distal section. The first connecting strut column can include individual first connecting struts and the second connecting strut column can include individual second connecting struts. The first connecting column couples the first and second expansion columns. Figure 1 shows an example where the first connecting column 32 couples the first expansion column 29 and the second expansion column 30. The second connecting column 33 couples the second expansion column 30 and the third expansion column 31.

Each expansion strut can have a stair-step configuration. Distal ends of expansion strut pairs of the first expansion column that are coupled to proximal ends of expansion strut pairs of the second expansion column can be vertically offset. Distal ends of expansion strut pairs of the second expansion column that are coupled to proximal ends of expansion strut pairs of the third expansion column can also be vertically offset.

Some embodiments of the stent include connecting struts with five sections, including an intermediate section, a proximal curvilinear section and a distal curvilinear section. The proximal section of each first connecting strut can be contralaterally conjoined to an expansion strut pair of the first expansion column. The distal section can be contralaterally conjoined to an expansion strut pair of the second expansion column. The proximal and distal sections can have the same lengths.

At least a portion of the proximal and distal curvilinear section can be parallel to a portion of an expansion strut pair loop in the first expansion column or in the second expansion column.

The proximal section can include a terminal end conjoined to an expansion strut in the first expansion column, and at least one surface that is conjoined to at least one surface of an expansion strut in the first expansion column. The distal section includes a terminal end conjoined to an expansion strut in the second expansion column, and at least one surface that is conjoined to an expansion strut in the second expansion column.

At least one of the proximal and distal sections of each connecting strut can be contralaterally conjoined to an expansion strut pair of the first and second expansion columns, or to an expansion pair of the second and third expansion columns.

At least a portion of the curvilinear proximal section of each connecting strut can be parallel to a portion of an expansion strut pair loop. At least a portion of the curvilinear distal section of each connecting strut can be parallel to a portion of an expansion strut pair loop of an expansion column.

At least a portion of the curvilinear proximal section of each connecting strut can be positioned in close proximity to an expansion strut pair loop of an expansion column. At least a portion of the curvilinear distal section of each connecting strut can be positioned in close proximity to an expansion strut pair loop. Close proximity can be in the range of 0.003 cm to 0.127 cm (0.001 to 0.050 of an inch), in the range of 0.003 cm to 0.102 cm (0.001 to 0.040 of an inch), or in the range of 0.003 cm to 0.076 cm (0.001 to 0.030 of an inch).

In various embodiments of the stent each connecting strut can have a proximal end, a distal end, four pivot points, and a longitudinal axis. The proximal end can extend in a first direction. The distal end of a connecting strut can extend in an second direction opposite to the first direction.

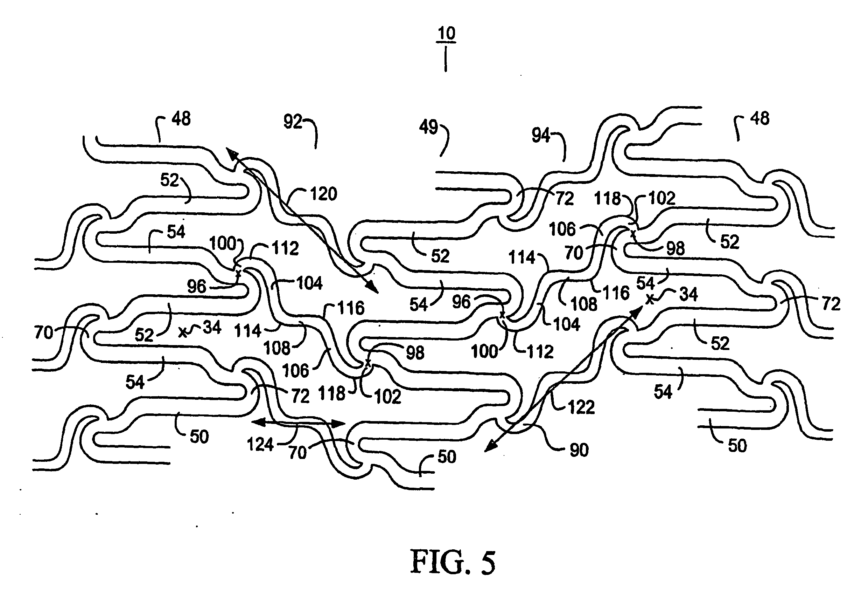

Each connecting strut in the first connecting strut column has two radii of curvature each with the proximal and distal curvilinear section. Figure 5 shows examples pivot points 112 and 114 having radii of curvature in the proximal curvilinear section, and pivot points 116 and 118 having radii of curvatures in the distal curvilinear section. Each pivot point can have at least one radius of curvature.

The longitudinal axis of the connecting strut may be non-parallel to a longitudinal axis of the stent. In some embodiments of the stent, each connecting strut in a same connecting strut column can share the similar longitudinal axis, which can be mutually parallel.

Each first connecting strut can have a longitudinal axis that extends in a first direction. Each second connecting strut can have a longitudinal axis that extends in an opposite second direction.

The intermediate section can have a longitudinal axis. The longitudinal axis of an intermediate section may be parallel to an expansion strut in the first expansion column, parallel to an expansion strut in the second expansion column, non-parallel to the longitudinal axis of the first connecting strut, and parallel to the longitudinal axis of the stent. The intermediate section is coupled to the curvilinear proximal section and the curvilinear distal section.

Each connecting strut is contralaterally conjoined to the first and second expansion columns. At least a portion of the connecting struts can have asymmetrical geometric configurations.

Some embodiments of the stent include a first end expansion column and a second end expansion column. The first and second end expansion columns can define a proximal and a distal end of the stent, and are mirror images of each other.

A plurality of cells can be defined by the first expansion column, the second expansion column and the first connecting strut column. Cells can have evenly spaced, asymmetrical geometric shapes. Cells can also have evenly spaced geometric shapes with a quasi-hexagonal geometry in a nominally expanded state.

Expansion strut pair loops in two adjacent expansion columns can be aligned in a peak-to-valley, in a valley-to-peak geometry, or in a peak-to-peak geometry.

An expansion column includes expansion struts that form expansion strut pairs in a ring shape. Each pair of expansion struts has two struts conjoined by a joining strut section at either a proximal or distal end. This pairing between two adjacent expansion struts conjoined by a joining strut section alternates from proximal to distal and distal to proximal. This sequence, in one embodiment, can continue twelve times seamlessly around the circumference of a ring for six zigzag cycles around the circumference of the ring shape. In such an embodiment there are twelve expansion strut pairs in alternating positions in an expansion column. There can also be twelve joining strut sections, six in a proximal end and six in a distal end in an alternating sequence. Expansion struts can include one or more of a short stepped-down segment at a proximal end, a short stepped-down segment at a distal end, a short stepped-up segment at a proximal end, and a short stepped-up segment at a distal end. Some embodiments of the stent include expansion struts with a short-sloped transitional segment between the long and short parts in the stair-step expansion struts. Various embodiments of the stent can include various combination of one or more of different expansion struts. At proximal or distal end of the stent, the terminating side of an end expansion column can have smooth and evenly rounded loops.

Various embodiments of the stent include one or more types of expansion columns. A first expansion column includes various expansion strut pairs. A joining strut section at a proximal end can join an expansion strut with a short stepped-down section at a proximal end and an expansion strut with a short stepped-down section at a distal end, forming an expansion strut pair loop. A joining strut section at a distal end can join an expansion strut with a short stepped-down section at a distal end and an expansion strut with a short stepped-down section at a proximal end, forming an expansion strut pair loop. These expansion strut pairs alternate, for example for six cycles, around the expansion ring without a break.

A second expansion column includes various expansion strut pairs. A joining strut section at a proximal end can join an expansion strut with a short stepped-up section at a proximal end and an expansion strut with a short stepped-up section at a distal end, forming an expansion strut pair loop. A joining strut section at a distal end can join an expansion strut with a short stepped-up section at a distal end and an expansion strut with a short stepped-up section at a proximal end, forming an expansion strut pair loop. These expansion strut pairs alternate, for example for six cycles, around the expansion ring without a break.

Different types of expansion columns can be arranged in an alternating sequence, inter-linked along the length of the stent by connecting columns. Stepped-up or stepped-down segments with a sloped transitional section can provide flexibility, smooth surface modulation effects, and well-formed crimping space to the stent. A connecting strut can conjoin with an expansion strut pair at a short stepped-down or stepped-up section of an expansion strut. A connecting strut can be a direct extension of an expansion strut and be integral to the stent structure rather than a separate structure added, welded, or attached. Separate terminology for stent elements, for example expansion and connecting struts, conveniently describing the anatomy and function of various stent portions.

Connecting struts can have a curvilinear double stair step shape with a longitudinal axis diagonally tilted to one side or the other side from the vertical, due to the diagonal orientation. Connecting struts of different connecting strut columns can have different longitudinal axes, which can be mirror images. In some embodiments of the stent, a connecting strut has three segments, two end-stem sections and four pivot points. Pivot points can have a varying radius of curvature. These multiple pivot points are responsible for flexibility. One end of a connecting strut can conjoin with an expansion strut pair in one expansion strut column and another end of the connecting strut can conjoin to another expansion strut pair in an adjacent expansion strut column. The connecting strut can link two apposing expansion strut pairs in a diagonal orientation. A diagonal orientation of a connecting strut of the stent gives added flexibility, excellent crimping, vessel conformability and smooth surface modulation to the stent.

Further, when a connecting strut conjoins expansion strut pairs, both ends of a connecting strut conjoin to the contralateral sides of apposing expansion strut pairs of adjacent expansion columns, at a stepped down or a stepped up sections. Conjoining a connecting strut on contralateral sides, along with a diagonal orientation and multiple pivot points, can provide good stent performance characteristics.

In some embodiments of the stent, the ratio of expansion strut to connecting strut number is two to one, where such as when a connecting strut is conjoined to expansion strut pairs.

When the expansion columns and connecting columns are conjoined, the stent can have a continuous, unbroken cylindrical form without breaks or de-linking around the circumference and along the length of the stent. Unbroken links between the expansion and connecting struts can make regular and evenly spaced asymmetrical cells. The cell size can be maximized or minimized by programming of the stent design platform, as dictated by clinical or applications requirements.

Figure 1 shows one embodiment of a stent 10 in side elevation view, with a first expansion column 29, a second expansion column 30, a third expansion column 31, a first connecting strut column 32, and a second connecting strut column 33. The stent 10 has a proximal end 20 and a distal end 22. The stent 10 has a tubular or cylindrical structure. The stent 10 has a longitudinal length 24 and a longitudinal axis 26.

In some embodiments of the stent, an expansion column can be a zigzag or corrugated ring configuration of expansion struts. An expansion column, for example expansion column 30, in a stent 10 can be an unbroken circular ring. Multiple expansion strut columns can be interconnected with connecting struts continuously along the longitudinal axis 26 of the stent 10 in an unbroken manner to form a stent 10 having a tubular shape. The interconnections among expansion columns and connecting strut columns enclose spaces, or cells, formed by expansion struts and connecting struts. In the embodiment shown in Figure 1, all cells have asymmetrical geometry. The stent 10 has two different diameters, including an outer diameter 36 and an inner diameter 38, having a difference of a thickness of the stent 10. Both the outer diameter 36 and inner diameter 38 can change as the stent 10 goes through a crimping stage, when the diameters 36 and 38 are narrowed, and through a deployed stage, when the diameters 36 and 38 are expanded.

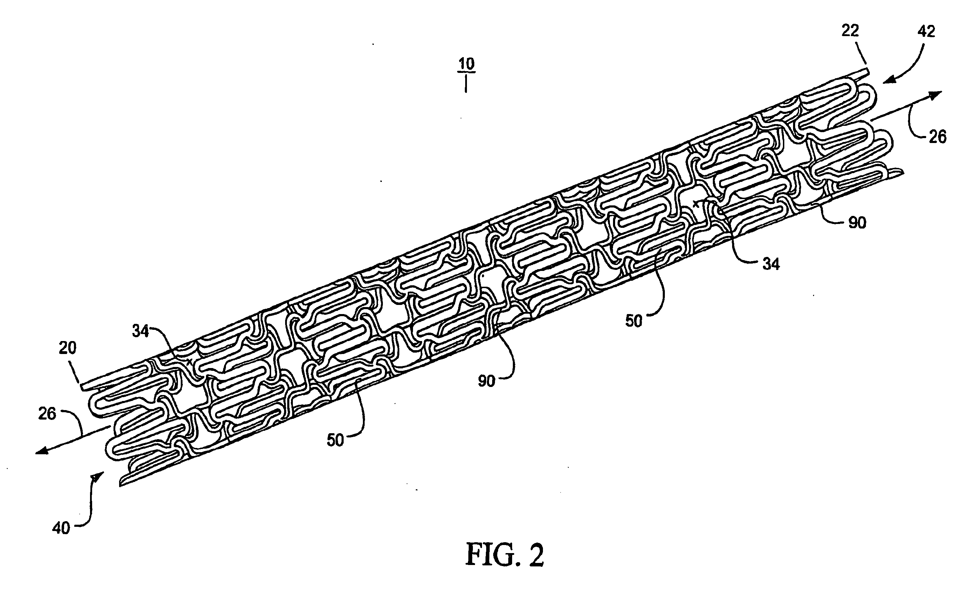

Figure 2 shows one embodiment of a stent 10 in isometric view. A back half of the stent 10 can be seen through the cell space of the front half of the stent 10. The shown embodiment of the stent 10 has a tubular structure with a central lumen, a proximal opening 40, and a distal opening 42. Stent cells 34 include open spaces in the network of expansion struts and connecting struts. The lumen includes the central, open tunnel formed by the expansion and connecting struts of the stent.

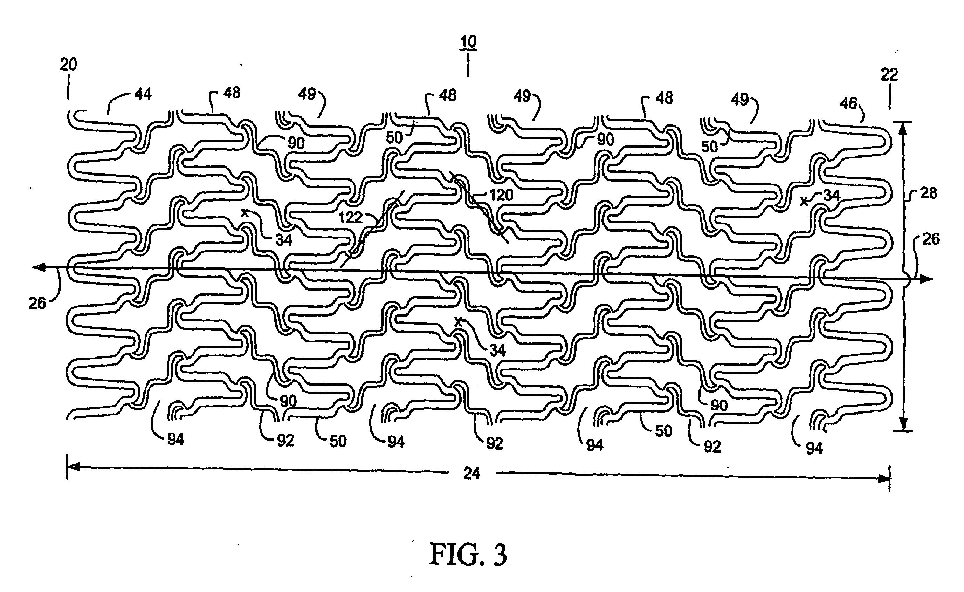

Figure 3 shows one embodiment of a stent 10 in cut-open 2-dimensional view. The stent 10 has a proximal end 20 and a distal end 22. This view of the stent 10 is a scale drawing for a 15 mm coronary stent. There are eight expansion columns and seven connecting strut columns. At the proximal end 20 is an expansion column 44, which is a mirror image of an expansion columns 46 at the distal end 22. In the middle of the stent 10, there are six expansion columns, such that an expansion column 49 alternates with an expansion column 48. Interconnecting with eight expansion columns along the longitudinal axis 26 of the stent 10 are seven connecting strut columns including four connecting strut columns 94 and three connecting strut columns 92, such that a connecting strut column 94 alternates with a connecting strut column 92. There are a total of 42 cells of various asymmetric configurations. All the cells in this embodiment have asymmetrical geometry. Expansion columns 44, 46, 48, and 49 are vertically arranged with expansion strut pair loops aligned peak-to-valley. Connecting strut columns 92 and 94 interconnect expansion columns 44, 46, 48, and 49 in a continuous and unbroken manner along the length 24 and around the circumference 28 of the stent 10.

The stent 10 in Figure 3 has the proximal end 20 on the left and the distal end 22 on the right. The stent 10 has a length 24 horizontally and a circumference 28 vertically, with a longitudinal axis 26 horizontally along the length 24 from the proximal end 20 to the distal end 22.

A width (horizontal dimension) of expansion columns is wider than a width of connecting strut columns. However, a width of a connecting strut column could be made the same or larger than a width of an expansion column. The variation of width ratio between a connecting strut column and an expansion column are within the scope of present invention of stent 10. The number of expansion strut cycles in an expansion column and the number of connecting struts in a connecting strut column can be made variably different. Variable numbers of making expansion strut cycles and connecting struts are within the scope of the present invention of the stent 10.

In some embodiments of the stent, one type of expansion column includes various expansion strut pairs. A joining strut section at a proximal end conjoins an expansion strut with short stepped-down section at a proximal end and an expansion strut with a short stepped-down section at a distal end, forming an expansion strut pair loop. A joining strut section at a distal end conjoins an expansion strut with a short stepped-down section at a distal end and an expansion strut with a short stepped-down section at a proximal end, forming an expansion strut pair loop. These expansion strut pairs alternate, for example for six cycles, around the expansion ring without a break.

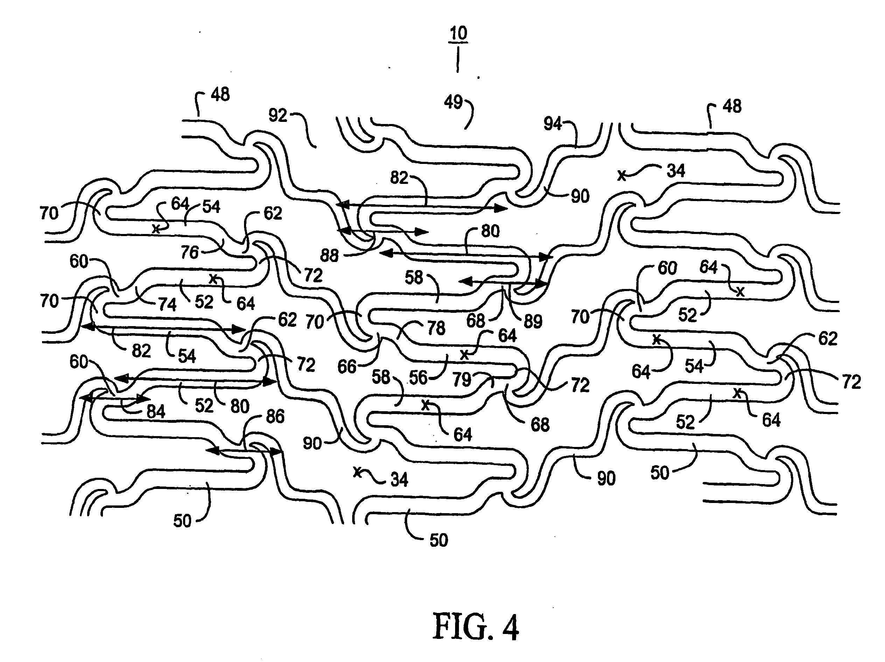

Figure 4 shows an embodiment having this type of expansion column 48. A joining strut section 70 at a proximal end conjoins an expansion strut 52 with a short stepped-down section at a proximal end and an expansion strut 54 with a short stepped-down section at a distal end forming an expansion strut pair loop. A joining strut section 72 at a distal end conjoins an expansion strut 54 with a short stepped-down section at a distal end 62 and an expansion strut 52 with a short stepped-down section at a proximal end 60, forming an expansion strut pair loop.

Another type of expansion column includes various expansion strut pair combinations. A joining strut section at a proximal end conjoins an expansion strut with a short stepped-up section at a proximal end and an expansion strut with a short stepped-up section at a distal end, forming an expansion strut pair loop. A joining strut section at a distal end conjoins an expansion strut with a short stepped-up section at a distal end and an expansion strut with a short stepped-up section at a proximal end, forming an expansion strut pair loop.

Figure 4 shows an embodiment having this type of expansion column 49. A joining strut section 70 at a proximal end conjoins an expansion strut 56 with a short stepped-up section at a proximal end 66 and an expansion strut 58 with a short stepped-up section at a distal end 68, forming an expansion strut pair loop. A joining strut section 72 at a distal end can join an expansion strut 58 with a short stepped-up section at a distal end 68 and an expansion strut 56 with a short stepped-up section at a proximal end 66, forming an expansion strut pair loop.

These proximal and distal expansion strut pairs alternate, for example for six cycles, around the expansion ring without a break.

A transitional slope 74 can be between a stepped down proximal section 60 and a straight section 64 in a stair step expansion strut 52. Likewise, a transitional slope 76 can be between a stepped down distal section 62 and a straight section 64 in a stair step expansion strut 54. A transitional slope 78 can be between a stepped up proximal section 66 and a straight section 64 in a stair step expansion strut 56. Likewise, a transitional slope 79 can be between a stepped up distal section 68 and a straight section 64 in a stair step expansion strut 58.

Figure 5 shows an example of connecting strut column 92 and connecting strut column 94. Connecting struts can have a curvilinear double stair step shape with a longitudinal axis is tilted to one side or the other side from the vertical plane, due to the diagonal orientation of connecting struts. Connecting struts of different connecting strut columns can have different longitudinal axes, which can be mirror images. For example, longitudinal axis 120 for connecting struts in connecting strut column 92 is different from longitudinal axis 122 for connecting struts in connecting strut column 94. In some embodiments of the stent, a connecting strut has three segments, two end-stem sections and four pivot points. Figure 5 shows connecting struts with a proximal curvilinear segment 104, central segment 108, distal curvilinear segment 106, proximal end-stem 100, distal end-stem 102, and pivot points 112, 114, 116, and 118. Pivot point 112 is a junction between proximal end-stem 100 and proximal curvilinear segment 104, pivot point 114 is a junction between proximal curvilinear segment 104 and central segment 108, pivot point 116 is a junction between central intermediate segment 108 and distal curvilinear segment 106, and pivot point 118 is a junction between distal curvilinear segment 106 and distal end-stem 102. These pivot points can have a varying degree of radius of curvature. These multiple pivot points are responsible for flexibility of the stent and for prevention of foreshortening of the stent. One end of a connecting strut can conjoin with an expansion strut pair in one expansion strut column and another end of the connecting strut can conjoin to another expansion strut pair in an adjacent expansion strut column. For example, a connecting strut in connecting strut column 92 has a proximal end 96 conjoined to an expansion strut in one expansion column, and a distal end 98 conjoined to an expansion strut in another expansion column. Proximal end 96 and distal end 98 of the connecting strut are conjoined to contralateral sides of apposing expansion strut pairs of adjacent expansion columns, at a stepped down or a stepped up sections. Conjoining a connecting strut on contralateral sides, along with a diagonal orientation and multiple pivot points of the connecting strut provides good stent performance characteristics. The connecting strut can link two apposing expansion strut pairs in a diagonal orientation. A diagonal orientation of a connecting strut of the stent gives added flexibility, excellent crimping, vessel conformability and smooth surface modulation to the stent.

## Claims

1. A stent in a non-expanded state, comprising:
- a first expansion column (29) including individual stair-step expansion struts forming a plurality of expansion strut pairs, each pair of expansion struts has two struts conjoined by a joining strut section at either a proximal or distal end, wherein the joining strut section is formed with loops that couple adjacent individual expansion struts, wherein two adjacent expansion strut pairs share a common strut, wherein one expansion strut of an expansion strut pair of the first expansion column (29) has a stair-step segment at a proximal segment and the other expansion strut of the expansion strut pair has a stair-step segment at a distal segment;
- a second expansion column (30) including individual stair-step expansion struts forming a plurality of expansion strut pairs, each pair of expansion struts has two struts conjoined by a joining strut section at either a proximal or distal end, wherein the joining strut section is formed with loops that couple adjacent individual expansion struts, wherein two adjacent expansion strut pairs share a common strut, wherein one expansion strut of an expansion strut pair of the second expansion column (30) has a stair-step segment at a distal segment and the other expansion strut of the expansion strut pair has a stair-step segment at a proximal segment;
- a first connecting strut column (32) including a plurality of individual first connecting struts that couple the first and second expansion columns,
wherein each of a first connecting strut in the first connecting strut column (32) has a curvilinear double stair-step shape and comprises three segments, wherein said first connecting strut includes a proximal curvilinear segment (104) a distal curvilinear segment (106) and an intermediate segment (108) having a stair step geometric configuration wherein both ends of the first connecting struts conjoin to contralateral sides of apposing expansion strut pairs of adjacent expansion columns at said stair-step segments of the expansion struts.

2. The stent of claims 1, wherein each first connecting strut in the first connecting strut column (32) has a proximal end that extends in a first direction and a distal end that extends in an opposite second direction.

3. The stent of one of the claims 1 or 2, wherein each first connecting strut in the first connecting strut column (32) has two end-stems (100, 102).

4. The stent of one of the claims 1 to 3, wherein each first connecting strut in the first connecting strut column (32) has four pivot points (112, 114, 116, 118).

5. The stent of claim 4, wherein each pivot point (112, 114, 116, 118) has at least one radius of curvature.

6. The stent of claims 1, wherein the intermediate segment (108) of each first connecting strut in the first connecting strut column (32) is coupled to the curvilinear proximal segment (104) and the curvilinear distal segment (106).

7. The stent of claims 1, wherein each first connecting strut in the first connecting strut column has a longitudinal axis that is non-parallel to a longitudinal axis of the stent.

8. The stent of claims 6 or 7, wherein each intermediate segment of each first connecting strut of the first connecting strut column (32) has a longitudinal axis that is non-parallel to the longitudinal axis of the first connecting strut.

9. The stent of claim 7, wherein each first connecting strut in the first connecting strut column (32) has the same longitudinal axis.

10. The stent of claim 7, wherein all of the first connecting struts in the first connecting strut column (32) have parallel longitudinal axes.

11. The stent of on of the claims 1 to 10, wherein each connecting strut in the first connecting strut column (32) has at least two radii of curvature at its proximal section and at least two radii of curvature at its distal section.

12. The stent of one of the preceding claims, wherein at least a portion of the first connecting struts of the first connecting strut column (32) have asymmetrical geometric configurations.

13. The stent of one of the preceding claims, wherein a terminal end (100) of the proximal segment (104) of each first connecting strut in the first connecting strut column (32) is conjoined to an expansion strut in the first expansion column (29), and a terminal end (102) of the distal section (106) of each first connecting strut is conjoined to an expansion strut in the second expansion column.

14. The stent of one of the preceding claims, wherein the proximal segment (104) of each first connecting strut has a surface that is conjoined to at least one surface of an expansion strut of an expansion strut pair in the first expansion column (29), and the distal segment (106) of each first connecting strut has at least one surface that is conjoined to an end of an expansion strut of an expansion strut pair in the second expansion column.

15. The stent of one of the preceding claims, wherein the proximal and distal segment (106, 108) of each first connecting strut of the first connecting strut column (32) have the same lengths.

16. The stent of one of the preceding claims, wherein at least a portion of the proximal segment (106) of each first connecting strut of the first connecting strut column (32) is in close proximity to an expansion strut pair of the first expansion column (29).

17. The stent of claim 16, wherein at least a portion of distal segment (108) of each first connecting strut of the first connecting strut column (32) is in close proximity to an expansion strut pair of the second expansion column (30).

18. The stent of one of the preceding claims, wherein distal ends of expansion strut pairs of the first expansion column (29) that are coupled to proximal ends of expansion strut pairs of the second expansion column (30) are vertically offset.

19. The stent of one of the preceding claims, further comprising: a third expansion column including individual expansion struts forming a plurality of expansion strut pairs with loops that couple adjacent individual expansion struts, wherein two adjacent expansion struts share a common strut; and a second connecting strut column including a plurality of individual connecting struts each with a proximal section and a distal section, wherein each of a second connecting strut in the second connecting strut column comprises three segment wherein said second connecting strut includes a proximal curvilinear section and a distal curvilinea segment and an intermediate segment having a stairstep geometric configuration.

20. The stent of one of the preceding claims, wherein at least a portion of the expansion strut pair loops has a radius of curvature.

21. The stent of claim 19, wherein at least a portion of the curvilinear proximal segment of each second connecting strut of the second connecting strut column is parallel to at least a portion of the radius of curvature of an expansion strut pair loop in the second expansion column.

22. The stent of claim 19, wherein at least a portion of the proximal segment of each second connecting strut of the second connecting strut column is in close proximity to an expansion strut pair loop of the second expansion column.

23. The stent of claim 19, wherein at least a portion of the distal segment of each second connecting strut of the second connecting strut column is in close proximity to an expansion strut pair loop of the second expansion column.

24. The stent of claim 19, wherein each first connecting strut of the first connecting strut column (32) has a longitudinal axis that extends in a first direction, and each second connecting strut of the second connecting strut column has a longitudinal axis that extends in an opposite second direction.

25. The stent of claim 19, wherein distal ends of expansion strut pairs of the second expansion column that are coupled to proximal ends of expansion strut pairs of the third expansion column are vertically offset.

26. The stent of claim 19, wherein expansion strut pair loops of the second and third expansion columns are aligned in a peak-to-valley geometry.

27. The stent of claim 19, wherein expansion strut pair loops of the second and first expansion columns are aligned in a valley-to-peak geometry.

28. The stent of one of the preceding claims further comprising: a first end expansion column and a second end expansion column, wherein the first and second end expansion columns are mirror images of each other.

29. The stent of claim 8, wherein each longitudinal axis of each intermediate segment of each first connecting strut in the first connecting strut column is parallel to the longitudinal axis of the stent.

30. The stent of one of the preceding claims, wherein at least a portion of the curvilinear proximal segment of each first connecting strut of the first connecting strut column is parallel to a portion of an expansion strut pair of the first expansion column.

31. The stent of claim 29, wherein at least a portion of the curvilinear distal segment of each first connecting strut of the first connecting strut column is in close proximity to an expansion strut pair of the second expansion column.

32. The stent of one of the preceding claims, wherein at least a portion of the curvilinear distal section of each first connecting strut of the first connecting strut column is parallel to a portion of an expansion strut pair of the second expansion column.

33. The stent of claim 19, wherein each first connecting strut of the first connecting strut column has a longitudinal axis that extends in a first direction, and each second connecting strut of the second connecting strut column has a longitudinal axis that extends in an opposite second direction.

34. The stent of claim 19, wherein distal ends of expansion strut pairs of the second expansion column that are coupled to proximal ends of expansion strut pairs of the third expansion column are vertically offset.

35. The stent of claim 19, wherein the plurality of cells have asymmetrical geometries.

36. The stent of claim 19, wherein the plurality of cells have evenly spaced geometric shapes with a quasi-hexagonal geometry in a nominally expanded state.

37. The stent of claims 16, 22 or 30, wherein close proximity is in the range of 0.003 cm to 0.127 cm (0.001 to 0.050 of an inch).

## Patentansprüche

1. Stent in nicht expandiertem Zustand, der umfasst:
- ein erstes säulenförmiges Expansionsglied (29), das einzelne treppenstufenförmige Expansionsstreben enthält, die eine Vielzahl von Expansionsstrebenpaaren bilden, wobei jedes Paar von Expansionsstreben zwei Streben besitzt, die durch einen verbindenden Strebenabschnitt entweder am nahen oder am fernen Ende verbunden sind, wobei der verbindende Strebenabschnitt mit Schlaufen ausgebildet ist, die benachbarte einzelne Expansionsstreben verbinden, wobei zwei benachbarte Expansionsstrebenpaare eine gemeinsame Strebe teilen, wobei eine Expansionsstrebe eines Expansionsstrebenpaares des ersten säulenförmigen Expansionsglieds (29) ein treppenstufenförmiges Segment an einem nahen Segment besitzt und die andere Expansionsstrebe des Expansionsstrebenpaares ein treppenstufenförmiges Segment an einem fernen Segment besitzt;
- ein zweites säulenförmiges Expansionsglied (30), das einzelne treppenstufenförmige Expansionsstreben enthält, die eine Vielzahl von Expansionsstrebenpaaren bilden, wobei jedes Paar von Expansionsstreben zwei Streben besitzt, die durch einen verbindenden Strebenabschnitt entweder am nahen oder am fernen Ende verbunden sind, wobei der verbindende Strebenabschnitt mit Schlaufen ausgebildet ist, die benachbarte einzelne Expansionsstreben verbinden, wobei zwei benachbarte Expansionsstrebenpaare eine gemeinsame Strebe teilen, wobei eine Expansionsstrebe eines Expansionsstrebenpaares des zweiten säulenförmigen Expansionsglieds (30) ein treppenstufenförmiges Segment an einem fernen Segment besitzt und die andere Expansionsstrebe des Expansionsstrebenpaares ein treppenstufenförmiges Segment an einem nahen Segment besitzt;
- ein erstes verbindendes säulenförmiges Verstrebungsglied (32), das eine Vielzahl von einzelnen ersten Verbindungsstreben enthält, die die ersten und zweiten säulenförmigen Expansionsglieder verbinden,
wobei jede der ersten Verbindungsstreben im ersten verbindenden säulenförmigen Verstrebungsglied (32) eine gekrümmte doppelte treppenstufenförmige Gestalt hat und drei Segmente aufweist, wobei die besagte erste Verbindungsstrebe ein nahes gekrümmtes Segment (104), ein fernes gekrümmtes Segment (106) und ein dazwischenliegendes Segment (108) mit einer treppenstufenförmigen geometrischen Struktur enthält, wobei sich beide Enden der ersten Verbindungsstreben mit gegenüberliegenden Seiten gegenüberliegender Expansionsstrebenpaare benachbarter säulenförmiger Expansionsglieder an den treppenstufenförmigen Segmenten der Expansionsstreben verbinden.

2. Stent nach Anspruch 1, wobei jede erste Verbindungsstrebe im ersten verbindenden säulenförmigen Verstrebungsglied (32) ein nahes Ende besitzt, das sich in eine erste Richtung erstreckt, und ein fernes Ende besitzt, das sich in eine entgegengesetzte zweite Richtung erstreckt.

3. Stent nach einem der Ansprüche 1 oder 2, wobei jede erste Verbindungsstrebe im ersten verbindenden säulenförmigen Verstrebungsglied (32) zwei Endstiele (100, 102) besitzt.

4. Stent nach einem der Ansprüche 1 bis 3, wobei jede erste Verbindungsstrebe im ersten verbindenden säulenförmigen Verstrebungsglied (32) vier Wendepunkte (112, 114, 116, 118) besitzt.

5. Stent nach Anspruch 4, wobei jeder Wendpunkt (112, 114, 116, 118) zumindest einen Krümmungsradius besitzt.

6. Stent nach Anspruch 1, wobei das dazwischenliegende Segment (108) einer jeden ersten Verbindungsstrebe im ersten verbindenden säulenförmigen Verstrebungsglied (32) mit dem gekrümmten nahen Segment (104) und dem gekrümmten fernen Segment (106) gekoppelt ist.

7. Stent nach Anspruch 1, wobei jede erste Verbindungsstrebe im ersten verbindenden säulenförmigen Verstrebungsglied eine Längsachse besitzt, die nicht parallel zur Längsachse des Stents verläuft.

8. Stent nach Anspruch 6 oder 7, wobei jedes dazwischenliegende Segment einer jeden ersten Verbindungsstrebe im ersten verbindenden säulenförmigen Verstrebungsglied (32) eine Längsachse besitzt, die nicht-parallel zur Längsachse der ersten Verbindungsstrebe verläuft.

9. Stent nach Anspruch 7, wobei jede erste Verbindungsstrebe im ersten verbindenden säulenförmigen Verstrebungsglied (32) die gleiche Längsachse besitzt.

10. Stent nach Anspruch 7, wobei alle ersten Verbindungsstreben im ersten verbindenden säulenförmigen Verstrebungsglied (32) parallel verlaufende Längsachsen besitzen.

11. Stent nach einem der Ansprüche 1 bis 10, wobei jede Verbindungsstrebe im ersten verbindenden säulenförmigen Verstrebungsglied (32) zumindest zwei Krümmungsradien an ihrem nahen Abschnitt und zumindest zwei Krümmungsradien an ihrem fernen Abschnitt aufweist.

12. Stent nach einem der vorhergehenden Ansprüche, wobei zumindest ein Teil der ersten Verbindungsstreben des ersten verbindenden säulenförmigen Verstrebungsglieds (32) asymmetrische geometrische Strukturen besitzt.

13. Stent nach einem der vorhergehenden Ansprüche, wobei ein abschließendes Ende (100) des nahen Segments (104) einer jeden ersten Verbindungsstrebe im ersten verbindenden säulenförmigen Verstrebungsglied (32) mit einer Expansionsstrebe im ersten säulenförmigen Expansionsglied (29) verbunden ist, und ein abschließendes Ende (102) des fernen Segments (106) einer jeden ersten Verbindungsstrebe mit einer Expansionsstrebe im zweiten säulenförmigen Expansionsglied verbunden ist.

14. Stent nach einem der vorhergehenden Ansprüche, wobei das nahe Segment (104) einer jeden ersten Verbindungsstrebe eine Oberfläche besitzt, die mit zumindest einer Oberfläche einer Expansionsstrebe eines Expansionsstrebenpaares im ersten säulenförmigen Expansionsglied (29) verbunden ist, und wobei das ferne Segment (106) einer jeden ersten Verbindungsstrebe zumindest eine Oberfläche besitzt, die mit einem Ende einer Expansionsstrebe eines Expansionsstrebenpaares im zweiten säulenförmigen Expansionsglied verbunden ist.

15. Stent nach einem der vorhergehenden Ansprüche, wobei das nahe Segment und das ferne Segment (106,108) einer jeden ersten Verbindungsstrebe des ersten verbindenden säulenförmigen Verstrebungsglieds (32) die gleichen Längen besitzen.

16. Stent nach einem der vorhergehenden Ansprüche, wobei zumindest ein Teil des nahen Segments (106) einer jeden ersten Verbindungsstrebe des ersten verbindenden säulenförmigen Verstrebungsglieds (32) sich in enger Nachbarschaft zu einem Expansionsstrebenpaar des ersten säulenförmigen Expansionsglieds (29) befindet.

17. Stent nach Anspruch 16, wobei zumindest ein Teil des fernen Segments (108) einer jeden ersten Verbindungsstrebe des ersten verbindenden säulenförmigen Verstrebungsglieds (32) sich in enger Nachbarschaft eines Expansionsstrebenpaares des zweiten säulenförmigen Expansionsglieds (30) befindet.

18. Stent nach einem der vorhergehenden Ansprüche, wobei die fernen Enden von Expansionsstrebenpaaren des ersten säulenförmigen Expansionsglieds (29), die mit den nahen Enden von Expansionsstrebenpaaren des zweiten säulenförmigen Expansionsglieds (30) gekoppelt sind, vertikal versetzt sind.

19. Stent nach einem der vorhergehenden Ansprüche, der ferner umfasst:
- ein drittes säulenförmiges Expansionsglied,
das einzelne Expansionsstreben enthält, die eine Vielzahl von Expansionsstrebenpaaren mit Schlaufen bilden, die benachbarte einzelne Expansionsstreben miteinander koppeln, wobei zwei benachbarte Expansionsstreben eine gemeinsame Strebe gemeinsam nutzen;
- und ein zweites verbindendes säulenförmiges Verstrebungsglied, das eine Vielzahl von einzelnen Verbindungsstreben enthält, jede mit einem nahen Abschnitt und einem fernen Abschnitt, wobei jede der zweiten Verbindungsstreben im zweiten verbindenden säulenförmigen Verstrebungsglied drei Segmente aufweist, und die besagte zweite Verbindungsstrebe ein nahes gekrümmtes Segment und ein fernes gekrümmtes Segment und ein dazwischenliegendes Segment mit einer treppenstufenförmigen geometrischen Struktur enthält.

20. Stent nach einem der vorhergehenden Ansprüche, wobei zumindest ein Teil der Expansionsstrebenpaar-Schlaufen einen Krümmungsradius besitzt.

21. Stent nach Anspruch 19, wobei zumindest ein Teil des gekrümmten nahen Segments einer jeden zweiten Verbindungsstrebe des zweiten verbindenden säulenförmigen Verstrebungsglieds parallel zumindest zu einem Teil des Krümmungsradius einer Expansionsstrebenpaar-Schlaufe im zweiten säulenförmigen Expansionsglied verläuft.

22. Stent nach Anspruch 19, wobei zumindest ein Teil des nahen Segments einer jeden zweiten Verbindungsstrebe des zweiten verbindenden säulenförmigen Verstrebungsglieds sich in enger Nachbarschaft eines Expansionsstrebenpaares mit Schlaufen des zweiten säulenförmigen Expansionsglieds befindet.

23. Stent nach Anspruch 19, wobei zumindest ein Teil des fernen Segments einer jeden zweiten Verbindungsstrebe des zweiten verbindenden säulenförmigen Verstrebungsglieds sich in enger Nachbarschaft eines Expansionsstrebenpaares mit Schlaufen des zweiten säulenförmigen Expansionsglieds befindet.

24. Stent nach Anspruch 19, wobei jede erste Verbindungsstrebe des ersten verbindenden säulenförmigen Verstrebungsglieds (32) eine Längsachse besitzt, die sich in eine erste Richtung erstreckt, und
jede zweite Verbindungsstrebe des zweiten verbindenden säulenförmigen Verstrebungsglieds eine Längsachse besitzt, die sich in eine entgegengesetzte zweite Richtung erstreckt.

25. Stent nach Anspruch 19, wobei die fernen Enden der Expansionsstrebenpaare des zweiten säulenförmigen Expansionsglieds, die mit den nahen Enden der Expansionsstrebenpaare des dritten säulenförmigen Expansionsglieds gekoppelt sind, vertikal versetzt sind.

26. Stent nach Anspruch 19, wobei die Expansionsstrebenpaare mit Schlaufen der zweiten und dritten säulenförmigen Expansionsglieder in einer Scheitelpunkt-zu-Tal-Geometrie ausgerichtet sind.

27. Stent nach Anspruch 19, wobei die Expansionsstrebenpaare mit Schlaufen der zweiten und dritten säulenförmigen Expansionsglieder in einer Tal-zu-Scheitelpunkt-Geometrie ausgerichtet sind.

28. Stent nach einem der vorhergehenden Ansprüche, der ferner umfasst: ein erstes abschließendes säulenförmiges Expansionsglied und ein zweites abschließendes säulenförmiges Expansionsglied, wobei die ersten und zweiten abschließenden säulenförmigen Expansionsglieder Spiegelbilder voneinander darstellen.

29. Stent nach Anspruch 8, wobei jede Längsachse eines jeden dazwischenliegenden Segments einer jeden ersten Verbindungsstrebe im ersten verbindenden säulenförmigen Verstrebungsglied parallel zur Längsachse des Stents verläuft.

30. Stent nach einem der vorhergehenden Ansprüche, wobei zumindest ein Teil des gekrümmten nahen Segments einer jeden ersten Verbindungsstrebe des ersten verbindenden säulenförmigen Verstrebungsglieds parallel zu einem Teil eines Expansionsstrebenpaares des ersten säulenförmigen Expansionsglieds verläuft.

31. Stent nach Anspruch 29, wobei zumindest ein Teil des gekrümmten fernen Segments einer jeden ersten Verbindungsstrebe des ersten verbindenden säulenförmigen Verstrebungsglieds sich in enger Nachbarschaft eines Expansionsstrebenpaares des zweiten säulenförmigen Expansionsglieds befindet.

32. Stent nach einem der vorhergehenden Ansprüche, wobei zumindest ein Teil des gekrümmten fernen Segments einer jeden ersten Verbindungsstrebe des ersten verbindenden säulenförmigen Verstrebungsglieds parallel zu einem Teil eines Expansionsstrebenpaares des zweiten säulenförmigen Expansionsglieds verläuft.

33. Stent nach Anspruch 19, wobei jede erste Verbindungsstrebe des ersten verbindenden säulenförmigen Verstrebungsglieds eine Längsachse besitzt, die sich in eine erste Richtung erstreckt, und jede zweite Verbindungsstrebe des zweiten verbindenden säulenförmigen Verstrebungsglieds eine Längsachse besitzt, die sich in eine entgegengesetzte zweite Richtung erstreckt.

34. Stent nach Anspruch 19, wobei die fernen Enden der Expansionsstrebenpaare des zweiten säulenförmigen Expansionsglieds, die mit den nahen Enden der Expansionsstrebenpaare des dritten säulenförmigen Expansionsglieds gekoppelt sind, vertikal versetzt sind.

35. Stent nach Anspruch 19, wobei die Mehrheit der Zellen eine asymmetrische Geometrie besitzt.

36. Stent nach Anspruch 19, wobei die Mehrheit der Zellen gleichmäßig beabstandete geometrische Formen mit einer quasi-hexagonalen Geometrie in einem nominal expandierten Zustand besitzt.

37. Stent nach den Ansprüchen 16, 22 oder 30, wobei eine enge Nachbarschaft in einem Bereich von 0.003 cm bis zu 0.127 cm (0.001 bis zu 0.050 inch) liegt.

## Revendications

1. Prothèse endovasculaire dans un état non expansé, comprenant :
- une première colonne d'expansion (29) comprenant des entretoises d'expansion en escalier individuelles formant une pluralité de paires d'entretoises d'expansion, chaque paire d'entretoises d'expansion ayant deux entretoises raccordées par une section d'entretoise de raccordement au niveau d'une extrémité proximale ou distale, dans laquelle la section d'entretoise de raccordement est formée par des boucles qui couplent les entretoises d'expansion individuelles adjacentes, dans laquelle deux paires d'entretoises d'expansion adjacentes partagent une entretoise commune, dans laquelle une entretoise d'expansion d'une paire d'entretoises d'expansion de la première colonne d'expansion (29) a un segment en escalier au niveau d'un segment proximal et l'autre entretoise d'expansion de la paire d'entretoises d'expansion a un segment en escalier au niveau d'un segment distal ;
- une seconde colonne d'expansion (30) comprenant des entretoises d'expansion en escalier individuelles formant une pluralité de paires d'entretoises d'expansion, chaque paire d'entretoises d'expansion ayant deux entretoises raccordées par une section d'entretoise de raccordement au niveau d'une extrémité proximale ou distale, dans laquelle la section d'entretoise de raccordement est formée par des boucles qui couplent les entretoises d'expansion individuelles adjacentes, dans laquelle deux paires d'entretoises d'expansion adjacentes partagent une entretoise commune, dans laquelle une entretoise d'expansion d'une paire d'entretoises d'expansion de la seconde colonne d'expansion (30) a un segment en escalier au niveau d'un segment distal et l'autre entretoise d'expansion de la paire d'entretoises d'expansion a un segment en escalier au niveau d'un segment proximal ;
- une première colonne d'entretoises de connexion (32) comprenant une pluralité de premières entretoises de connexion individuelles qui couplent la première et seconde colonnes d'expansion,
dans laquelle chacune d'une première entretoise de connexion dans la première colonne d'entretoises de connexion (32) a une forme en escalier double curviligne et comprend trois segments, dans laquelle ladite première entretoise de connexion comprend un segment proximal curviligne (104), un segment distal curviligne (106) et un segment intermédiaire (108) ayant une configuration géométrique en escalier, dans laquelle les deux extrémités des premières entretoises de connexion se raccordent à des côtés controlatéraux des paires d'entretoises d'expansion opposées des colonnes d'expansion adjacentes au niveau desdits segments en escalier des entretoises d'expansion.

2. Prothèse endovasculaire selon la revendication 1, dans laquelle chaque première entretoise de connexion dans la première colonne d'entretoises de connexion (32) a une extrémité proximale qui s'étend dans une première direction et une extrémité distale qui s'étend dans une seconde direction opposée.

3. Prothèse endovasculaire selon les revendications 1 ou 2, dans laquelle chaque première entretoise de connexion dans la première colonne d'entretoises de connexion (32) a destiges à deux extrémités (100, 102).

4. Prothèse endovasculaire selon les revendications 1 à 3, dans laquelle chaque première entretoise de connexion dans la première colonne d'entretoises de connexion (32) a quatre points pivotants (112, 114, 116, 118).

5. Prothèse endovasculaire selon la revendication 4, dans laquelle chaque point pivotant (112, 114, 116, 118) a au moins un rayon de courbure.

6. Prothèse endovasculaire selon la revendication 1, dans laquelle le segment intermédiaire (108) de chaque première entretoise de connexion dans la première colonne d'entretoises de connexion (32) est couplé au segment proximal curviligne (104) et au segment distal curviligne (106).

7. Prothèse endovasculaire selon la revendication 1, dans laquelle chaque première entretoise de connexion dans la première colonne d'entretoises de connexion a un axe longitudinal qui n'est pas parallèle à un axe longitudinal de la prothèse endovasculaire.

8. Prothèse endovasculaire selon la revendication 6 ou 7, dans laquelle chaque segment intermédiaire de chaque première entretoise de connexion de la première colonne d'entretoises de connexion (32) a un axe longitudinal qui n'est pas parallèle à l'axe longitudinal de la première entretoise de connexion.

9. Prothèse endovasculaire selon la revendication 7, dans laquelle chaque première entretoise de connexion dans la première colonne d'entretoises de connexion (32) a le même axe longitudinal.

10. Prothèse endovasculaire selon la revendication 7, dans laquelle toutes les premières entretoises de connexion dans la première colonne d'entretoises de connexion (32) ont des axes longitudinaux parallèles.

11. Prothèse endovasculaire selon l'une des revendications 1 à 10, dans laquelle chaque entretoise de connexion dans la première colonne d'entretoises de connexion (32) a au moins deux rayons de courbure au niveau de sa section proximale et au moins deux rayons de courbure au niveau de sa section distale.

12. Prothèse endovasculaire selon l'une des revendications précédentes, dans laquelle au moins une partie des premières entretoises de connexion de la première colonne d'entretoises de connexion (32) ont des configurations géométriques asymétriques

13. Prothèse endovasculaire selon l'une des revendications précédentes, dans laquelle une extrémité terminale (100) du segment proximal (104) de chaque première entretoise de connexion dans la première colonne d'entretoises de connexion (32) est raccordée à une entretoise d'expansion dans la première colonne d'expansion (29), et une extrémité terminale (102) de la section distale (106) de chaque première entretoise de connexion est raccordée à une entretoise d'expansion dans la seconde colonne d'expansion.

14. Prothèse endovasculaire selon l'une des revendications précédentes, dans laquelle le segment proximal (104) de chaque première entretoise de connexion a une surface qui est raccordée à au moins une surface d'une entretoise d'expansion d'une paire d'entretoises d'expansion dans la première colonne d'expansion (29), et le segment distal (106) de chaque première entretoise de connexion a au moins une surface qui est raccordée à une extrémité d'une entretoise d'expansion d'une paire d'entretoises d'expansion dans la seconde colonne d'expansion.

15. Prothèse endovasculaire selon l'une des revendications précédentes, dans laquelle le segment proximal et le segment distal (106, 108) de chaque première entretoise de connexion de la première colonne d'entretoises de connexion (32) ont les mêmes longueurs.

16. Prothèse endovasculaire selon l'une des revendications précédentes, dans laquelle au moins une partie du segment proximal (106) de chaque première entretoise de connexion de la première colonne d'entretoises de connexion (32) est proche d'une paire d'entretoises d'expansion de la première colonne d'expansion (29).

17. Prothèse endovasculaire selon la revendication 16, dans laquelle au moins une partie du segment distal (108) de chaque première entretoise de connexion de la première colonne d'entretoises de connexion (32) est proche d'une paire d'entretoises d'expansion de la seconde colonne d'expansion (30).

18. Prothèse endovasculaire selon l'une des revendications précédentes, dans laquelle les extrémités distales des paires d'entretoises d'expansion de la- première colonne d'expansion (29) qui sont couplées aux extrémités proximales des paires d'entretoises d'expansion de la seconde colonne d'expansion (30) sont décalées verticalement.

19. Prothèse endovasculaire selon l'une des revendications précédentes, comprenant en outre:
une troisième colonne d'expansion comprenant des entretoises d'expansion individuelles formant une pluralité de paires d'entretoises d'expansion avec des boucles qui couplent les entretoises d'expansion individuelles adjacentes, dans laquelle deux entretoises d'expansion adjacentes partagent une entretoise commune ; et une seconde colonne d'entretoises de connexion comprenant une pluralité d'entretoises de connexion individuelles chacune avec
une section proximale et une section distale, dans laquelle chacune d'une seconde entretoise de connexion dans la seconde colonne d'entretoises de connexion comprend trois segments, dans laquelle ladite seconde entretoise de connexion comprend une section proximale curviligne et un segment distal curviligne et un segment intermédiaire ayant une configuration géométrique en escalier.

20. Prothèse endovasculaire selon l'une des revendications précédentes, dans laquelle au moins une partie des boucles de la paire d'entretoises d'expansion a un rayon de courbure.

21. Prothèse endovasculaire selon la revendication 19, dans laquelle au moins une partie du segment proximal curviligne de chaque seconde entretoise de connexion de la seconde colonne d'entretoises de connexion est parallèle à au moins une partie du rayon de courbure d'une boucle de paire d'entretoises d'expansion dans la seconde colonne d'expansion.

22. Prothèse endovasculaire selon la revendication 19, dans laquelle au moins une partie du segment proximal de chaque seconde entretoise de connexion de la seconde colonne d'entretoises de connexion est proche d'une boucle de paire d'entretoises d'expansion de la seconde colonne d'expansion.

23. Prothèse endovasculaire selon la revendication 19, dans laquelle au moins une partie du segment distal de chaque entretoise de connexion de la seconde colonne d'entretoises de connexion est proche d'une boucle de paire d'entretoises d'expansion de la seconde colonne d'expansion.

24. Prothèse endovasculaire selon la revendication 19, dans laquelle chaque première entretoise de connexion de la première colonne d'entretoise de connexion (32) a un axe longitudinal qui s'étend dans une première direction, et chaque seconde entretoise de connexion de la seconde colonne d'entretoises de connexion a un axe longitudinal qui s'étend dans une seconde direction opposée.

25. Prothèse endovasculaire selon la revendication 19, dans laquelle les extrémités distales des paires d'entretoises d'expansion de la seconde colonned'expansion qui sont couplées aux extrémités proximales des paires d'entretoises d'expansion de la troisième colonne d'expansion sont décalées verticalement.

26. Prothèse endovasculaire selon la revendication 19, dans laquelle les boucles des paires d'entretoises d'expansion de la seconde et de la troisième colonnes d'expansion sont alignées en une géométrie de crête à creux.

27. Prothèse endovasculaire selon la revendication 19, dans laquelle les boucles des paires d'entretoises d'expansion de la seconde et de la première colonnes d'expansion sont alignées en une géométrie de creux à crête.

28. Prothèse endovasculaire selon l'une des revendications précédentes, comprenant en outre: une première colonne d'expansion d'extrémité et une seconde colonne d'expansion d'extrémité, dans laquelle la première et la seconde colonnes d'expansion d'extrémité sont l'image inversée l'une de l'autre.

29. Prothèse endovasculaire selon la revendication 8, dans laquelle chaque axe longitudinal de chaque segment intermédiaire de chaque première entretoise de connexion dans la première colonne d'entretoises de connexion est parallèle à l'axe longitudinal de la prothèse endovasculaire.

30. Prothèse endovasculaire selon l'une des revendications précédentes, dans laquelle au moins une partie du segment proximal curviligne de chaque première entretoise de connexion de la première colonne d'entretoises de connexion est parallèle à une partie d'une paire d'entretoises d'expansion de la première colonne d'expansion.

31. Prothèse endovasculaire selon la revendication 29, dans laquelle au moins une partie du segment distal curviligne de chaque première entretoise de connexion de la première colonne d'entretoises de connexion est proche d'une paire d'entretoises d'expansion de la seconde colonne d'expansion.

32. Prothèse endovasculaire selon l'une des revendications précédentes, dans laquelle au moins une partie de la section distale curviligne de chaque première entretoise de connexion de la première colonne d'entretoises de connexion est parallèle à une partie d'une paire d'entretoises d'expansion de la seconde colonne d'expansion.

33. Prothèse endovasculaire selon la revendication 19, dans laquelle chaque première entretoise de connexion de la première colonne d'entretoises de connexion a un axe longitudinal qui s'étend dans une première direction, et chaque seconde entretoise de connexion de la seconde colonne d'entretoises de connexion a un axe longitudinal qui s'étend dans une seconde direction opposée.

34. Prothèse endovasculaire selon la revendication 19, dans laquelle les extrémités distales des paires d'entretoises d'expansion de la seconde colonne d'expansion qui sont couplées aux extrémités proximales des paires d'entretoises d'expansion de la troisième colonne d'expansion sont décalées verticalement.

35. Prothèse endovasculaire selon la revendication 19, dans laquelle les plusieurs cellules ont des géométries asymétriques.

36. Prothèse endovasculaire selon la revendication 19, dans laquelle les plusieurs cellules ont des formes géométriques espacées de manière constante, avec une géométrie quasi-hexagonale dans un état nominalement expansé.

37. Prothèse endovasculaire selon la revendication 16, 22 ou 30, dans laquelle la proximité est dans la plage de 0,003 cm à 0,127 cm (0,001 à 0,050 pouce).
